# FASCICULE DE BREVET EUROPEEN

(11) **EP 1 456 247 B1**
(45) Date de publication et mention de la délivrance du brevet: **29.08.2007**
(21) Numéro de dépôt: 02803847.9
(22) Date de dépôt: 28.11.2002
(51) Int. Cl.: C08B 37/00, C08B 37/02, C07C 303/00, C07C 309/00, C07B 45/00

(54) **PROCEDE DE SULFONATION DE COMPOSES COMPRENANT DES GROUPEMENTS HYDROXYLES (OH) LIBRES OU DES AMINES PRIMAIRES OU SECONDAIRES**
VERFAHREN ZUR SULFONIERUNG VON VERBINDUNGEN MIT FREIEN HYDROXYLGRUPPEN (OH-GRUPPEN) ODER PRIMÄREN ODER SEKUNDÄREN AMINEN
METHOD FOR SULPHONATION OF COMPOUNDS COMPRISING FREE HYDROXYL (OH) GROUPS OR PRIMARY OR SECONDARY AMINES

(30) Priorité: 29.11.2001 FR 0115444
(43) Date de publication de la demande: 15.09.2004
(73) Titulaire: Organes, Tissus : Régénération, Réparation, Remplacement-OTR3, 94000 Créteil (FR)
(72) Inventeur: PETIT, Emmanuel, F-80000 Amiens (FR); GARCIA-PAPY, Dulce, F-91420 Morangis (FR); BARBIER-CHASSEFIERE, Véronique, F-91190 Gif sur Yvette (FR)
(74) Mandataire: Breese, Pierre
(86) Numéro de dépôt international: PCT/FR2002/004095
(87) Numéro de publication internationale: WO 2003/046014

(56) Documents cités:
- WO-A-99/29734
- US-A- 5 229 504

## Description

La présente invention a pour objet un procédé de préparation de composés sulfatés, dans des conditions, non dégradantes, reproductibles compatibles avec des utilisations desdits composés où une définition moléculaire précise doit être décrite, comme par exemples en tant qu'agents thérapeutiques.

Le procédé de l'invention s'applique à tout composé monomérique, oligomérique ou polymérique comprenant des groupements hydroxyle (OH) libres ou des amines primaires ou secondaires substituées ou non. Plus particulièrement, l'invention se rapporte à un procédé de O et N sulfonation, de polymères fonctionnalisés.

Les procédés de préparation de composés sulfatés rapportés dans l'art antérieur induisent généralement des coupures des chaînes des polymères lors de la O ou N sulfonation et donc la formation de résidus réactionnels potentiellement toxiques. Le procédé selon la présente invention vise précisément à éviter cet inconvénient en permettant l'addition contrôlée de groupements sulfonates dans des conditions ménagées telles que l'intégrité structurale du polymère initial, comme un oligosaccharide, ne soit pas altérée.

On connaît dans l'art antérieur des polymères fonctionnalisés aux propriétés anticoagulantes et notamment une famille de dérivés obtenus par substitutions, sur une chaîne de dextranes (D), des groupements carboxyméthyliques (CM), méthyle-benzylamide (B) et méthyle-benzylamidesulfonate (S), désignés sous l'abréviation CMDBS. Les procédés de préparation de ces polymères ont été décrits en particulier dans le brevet Français publié sous le No. 2 461 724 ainsi que dans le brevet US publié sous le No. 4 740 594. Aucun de ces brevets ne fournit une analyse précise des structures polymèriques obtenues et les procédés décrits ne permettent pas d'apporter l'évidence que les produits obtenus sont homogènes. Les conditions expérimentales de la réaction de sulfonation décrites dans ces brevets privilégient la fixation des sulfones sur les groupements OH libres du résidu glucosidique, et l'analyse présentée ne permet pas de mettre clairement en évidence la présence de benzylamine sulfonate.

D'autres membres de la famille des CMDBS, désignés HBGF pour "Heparin Binding Growth Factor", ont été décrits comme agents de cicatrisation dans le brevet US publié sous le N° 5693625. Les HBFGPP sont aussi décrits pour leurs propriétés de stimulations de la réparation et de la régénération des lésions induites dans les tissus musculaires (Brevet français No. 2 718 024), nerveux (Brevet français No. 2 718 026), du tractus digestif (Brevet français No. 2 718 023) ainsi que pour leur propriété anti-inflammatoire (Brevet français No. 2 718 025). Ces brevets établissent une série de critères fonctionnels permettant de cribler parmi tous les polymères biocompatibles ceux qui répondent aux quatre propriétés fonctionnelles suivantes :
- Protéger les facteurs de croissance à affinité pour l'héparine (HBGF) comme les facteurs de croissance des fibroblastes tels les FGF1&2 ou les facteurs transformants, comme le TGF betal, contre les dégradations protéolytiques ainsi que de potentialiser leurs activités biologiques dans une série de tests sur culture de cellules.
- Présenter une activité anticoagulante inférieure à 10 unités internationales par mg.
- Inhiber l'activité de l'élastase leucocytaire dans des conditions physiologiques.
- Inhiber l'activité de la plasmine dans des conditions physiologiques.

La demande de brevet Français No. 9809309 rapporte la structure d'une famille de polymères, désignés RGTA pour « ReGeneraTing Agent », présentant des propriétés des HBGFPP et décrit leur procédé de préparation et propriétés. Les RGTA présentent des effets antifibrotiques, notamment permettant d'améliorer la qualité des cicatrices cutanées, des effets anti-oxydants notamment pour traiter les effets délétères des radicaux libres (après des radiations ionisantes ou lors du stress oxydatif induit par ischémies) et des propriétés de régulation de l'homéostasie tissulaire notamment des masses osseuses. Ces propriétés complètent celles décrites pour les HBGFPP.

Les polymères décrits dans le brevet français No. 98 09309 répondent à la formule suivante :

AaXxYy (I)

dans laquelle :
A est un monomère ; X représente un groupement RCOOR' ; Y représente un groupement O ou N-sulfonate fixé sur A et répondant à l'une des formules suivante ROS03R', RNS03R' ; les groupements R sont des chaînes hydrocarbonées aliphatiques, éventuellement ramifiées et/ou insaturées et qui peuvent contenir un ou plusieurs cycles aromatiques à l'exception de la benzylamine et de la benzylamine sulfonate ; R' représente un atome d'hydrogène ou un cation ; a représente le nombre de monomères ; x représente le taux ou degré de substitution des monomères A par des groupements X ; y représente le taux ou degré de substitution des monomères A par des groupements Y

Parmi les polymères décrits dans le brevet français No. 98 09309, on peut citer des dérivés de dextrane comme les CMDS. Des polymères de type CMDS ont également été décrits comme anticoagulants (Maiga et al., Carbohydrate Polymers, 1997, 32, 89-93)

Dans tous ces exemples de l'art antérieur, les méthodes de synthèses décrites ne permettent pas l'obtention de produits selon les critères de reproductibilité suffisante assurant le maintien de l'intégrité moléculaire et l'absence de contaminants. En effet, si les procédés de substitutions des groupements carboxyliques sont largement décrits dans l'art antérieur et permettent la substitution contrôlée et ménagée assurant une reproductibilité suffisante, les procédés de sulfonation sont plus difficiles à maîtriser.

Les procédés de sulfonation sont réalisés à un pH très acide ce qui ne permet pas de préserver l'intégrité de la chaîne du polymère notamment si celle-ci est constituée de sucres naturels. De plus, ces conditions de sulfonation entraînent des décarboxylations difficiles à contrôler.

Ainsi de nombreux travaux de l'art antérieur décrivent des méthodes permettant de sulfater des polysaccharides de type Aa. Par exemple le dextrane sulfate (ou O-sulfonate) (DS), le carboxyméthyle-dextrane-sulfate (CM-D-S) (ou O-sulfonate), ou d'autres oligosaccharides sulfatés (ou O-sulfonaté) comme le xylane ou l'amidon ont été sulfatés (ou O-sulfonaté) par des procédés du type de celui décrit dans le brevet US No. 4 814437. Parmi ces méthodes, celles proposant une sulfonation de polysaccharides utilisant des acides forts comme agents de sulfonation ont été reportés par de nombreux auteurs. Par exemple, les brevets US No. 4,740,594 et No. 4,755,379 et Français No. 2 772 382 décrivent un traitement avec l'acide chlorosulfonique en utilisant le dichlorométhane (CH₂Cl₂) comme solvant pour la production de molécules de type CM-D-S et CM-D-B-S. Ce procédé était déjà largement décrit dans des travaux anciens puisque le même acide avait été utilisé pour la synthèse du DS avec de la pyridine comme solvant de caractère basique (Ricketts, Biochem J. 51, 210-133, 1952). L'acide sulfurique et l'acide sulfonique en présence de la formamide ont aussi été utilisés pour la préparation de DS à basses températures, comme décrit dans les brevets US No. 3 498 972 et 3 141 014. Il est connu que ces conditions de réaction fortement acides provoquent la dégradation partielle des produits même en présence de solvants de caractère basique ou à faibles températures. Elles entraînent une importante fragmentation de la chaîne macromoléculaire dans le cas de produits polymériques et une hydrolyse partielle de certains groupements fonctionnels contenus dans les molécules à sulfonater. Plusieurs améliorations à ces conditions de forte acidité ont été apportées avec l'utilisation de milieux mieux tamponnés ou d'acidités moindres. Ainsi, l'utilisation des complexes de trioxyde de soufre (SO₃) comme agents de sulfonation moins sévères que les acides sulfuriques et sulfoniques ont déjà été introduits (Archives of biochemistry and biophysics, 95, 36-41 ,1961 ; tetrahedron Letters, 29, 7, 803-806, 1988 ; J. Chem. Soc. Perkin trans. 1, 157, 1995). Plusieurs complexes de type SO₃-amine sont commercialisés, comme le SO₃-ME₃N (triméthylamine), le SO₃-Et₃N (triéthylamine), le SO₃-Pyridine, et le SO₃-Piperidine. Ces réactifs sont utilisés en milieu anhydre dans des solvants comme la DMF, la formamide et le DMSO. Cependant ces méthodes bien qu'utilisées pour la production des polysaccharides sulfatés, notamment comme ceux dérivés du dextrane, fonctionnalisés par des groupements ester, éther ou amide, présentent des inconvénients majeurs car ils entraînent :,
- la fragmentation aléatoire de la chaîne macromoléculaire des polymères, cependant dans des proportions moins importantes qu'avec les acides sulfoniques,
- une hydrolyse partielle des groupements fonctionnels déjà présents sur les polymères cités ci-dessus, et
- génèrent des produits secondaires par formation d'amines qui contaminent les préparations et qui se sont révélées toxiques après inoculation *in vivo* (Brain Res 16, 208-2, 473-478, 1981).

Ces techniques se sont encore améliorées afin d'éliminer au maximum les résidus toxiques ainsi que le propose pour la pyridine le procédé décrit dans le brevet US No. 4 814 437. En effet, les S03-amines utilisées dans ce procédé de l'art antérieur sont réactives avec les groupements aldéhydes présents sur l'extrémité réductrice de la plupart des polysaccharides, notamment dans le dextrane, et la liaison ainsi formée est covalente et donc permanente.

L'inconvénient de l'utilisation de complexes de type SO₃-amine a été également surmonté en employant des complexes de type SO₃-amide comme le SO₃-DMF et le SO₃-FA. Par exemple, les sels du DS, plus particulièrement les sels de sodium, sont actuellement préparés comme décrit dans le brevet US No. 4 855 416, par l'O-sulfonation du dextrane par un complexe de SO₃ associé à la formamide (SO₃-FA) avec de la formamide comme solvant. Cette méthode comprend la formation *in situ* du complexe SO₃-FA et puis sa réaction avec le dextrane. La forte réactivité du complexe SO₃-FA provoque une forte acidité du milieu réactionnel malgré l'utilisation d'une atmosphère inerte (N₂) et des solvants anhydres. Ce fait entraîne également la dégradation de la chaîne macromoléculaire.

Un autre complexe non-aminé très utilisé est le SO₃-DMF Il a aussi été proposé dans le brevet Français No. 2 772 382 pour la synthèse de polysaccharides sulfatés de type CM-D-S et CM-D-B-S. Cependant, le complexe SO₃-DMF comme le complexe SO₃-FA, produisent une forte acidité du milieu réactionnel, ce qui génère également la fragmentation de la chaîne macromoléculaire et l'hydrolyse de groupements labiles entraînant une perte du contrôle de la synthèse et des produits finaux. Ce procédé est donc aussi imparfait que les autres procédés dans le cas de la préparation de polymères poly-anioniques à hauts poids moléculaires que l'on ne souhaite pas couper d'une manière aléatoire et non contrôlée.

Le document US 5,229,504 décrit un procédé pour préparer du chitosane sulfonaté, en particulier par réaction d'un complexe SO3-DHF avec du Chitosane prétraité.

C'est dans ce souci d'amélioration des méthodes de synthèse, que des protocoles permettant l'obtention CMDBS et le CMDS ont été décrits dans le brevet Français No. 97 15702. Selon le brevet Français No. 97 15702, cette amélioration permet d'une part l'obtention de dérivés de dextrane présentant une plus grande homogénéité de poids moléculaire et d'autre part de contrôler les taux de substitution assurant une meilleure homogénéité des structures et ainsi une meilleure définition. Or, la méthode décrite dans le brevet Français No. 97 15702 repose, comme dans l'art antérieur, sur la sulfonation des polysaccharides utilisant du SO₃-amine (DMF, pyridine ou triethylamine), et les exemples donnés sont exclusivement obtenus avec la SO₃-Pyridine. En outre, cette méthode conduit à la formation de traces résiduelles de pyridine dont la toxicité pour l'homme est bien connue et ne semble pas permettre l'absence de formation de fragments de la chaîne du polysaccharide.

La présente invention a précisément pour but d'offrir de nouvelles méthodes générales de sulfonation permettant de contrôler avec beaucoup de rigueur et de précision les conditions des substitutions des groupements sulfonates sur des composés contenant des fonctions hydroxyles ou des fonctions amine primaires ou secondaires.

Ce but est atteint grâce à un procédé de sulfonation de composé présentant une ou plusieurs fonctions hydroxyles libres et/ou une ou plusieurs fonctions amines primaires ou secondaires substituées ou non, caractérisé en ce qu'il comprend le traitement dudit composé avec le complexe SO₃-DMF en présence d'un capteur d'acide, choisi dans le groupe comprenant : les alkènes ou alkines à point d'ébullition inférieur à 100°C ou un mélange de ceux-ci.

On entend par capteur d'acide une substance ou un mélange de substances capable de réagir sélectivement avec les protons libres en solution. Après l'addition de ce capteur d'acide au mélange réactionnel, les protons sont piégés par le capteur d'acide et ne participent plus à la baisse du pH car ils ne sont plus réactifs.

Avantageusement, le capteur d'acide est un butène comme le 2-méthyle-2-butène (2M2B), le 2-méthyle-propène, le 2-méthyle-pentène, ou leurs isomères, ou un mélange de ceux-ci.

Le procédé selon l'invention est remarquable en ce qu'il permet d'éviter la mise en oeuvre de pH trop acides et ainsi de supprimer le risque de coupures des composés traités. En outre, il présente l'avantage de ne pas introduire de substances toxiques difficiles ou impossibles à éliminer complètement.

Plus particulièrement, le procédé selon l'invention comprend les étapes suivantes :
a) la solubilisation ou la préparation d'une solution homogène du composé à sulfonater dans un solvant ou un co-solvant anhydre, comme la diméthylformamide (DMF) ou un co-solvant composé de formamide et de diméthylformamide.
b) l'addition à température ambiante (20-22 °C) d'un excès molaire d'un capteur d'acide, comme le 2-méthyle-2-butène, miscible dans le co-solvant.
c) l'addition rapide du complexe SO₃-DMF sous agitation,
d) l'agitation du mélange obtenu à l'étape précédente pendant une à deux heures à une température contrôlée et inférieure à 30 °C,
e) l'arrêt de la réaction par addition progressive du mélange sur une solution alcaline, par exemple une solution à 2 % de bicarbonate acide de soude (NaHCO₃) ou un autre alcali, avec un contrôle de pH jamais inférieur à 4 afin d'obtenir les sels du composé à sulfonater.

Le procédé de l'invention comprend avantageusement la purification du composé sulfonaté obtenu à l'étape (e), par exemple, par ultrafiltration tangentielle contre de l'eau (elle même de qualité injectable chez l'homme) à travers une membrane d'ultrafiltration à un seuil de coupure de 1000 Dalton.

De préférence, le solvant mis en oeuvre à l'étape (a) n'est pas le Diméthylsulfoxide (DMSO), car les travaux réalisés dans le cadre de la présente invention ont mis en évidence que ce solvant est très difficilement éliminable des préparations de polysaccharides sulfonatés et donc constitue un obstacle pour leur utilisation dans la préparation de formes pharmaceutiques.

Lorsque le composé à sulfonater est peu soluble dans un solvant ou un co-solvant anhydre une forme particulière de mise en oeuvre du procédé consiste à protoner ledit composé afin de favoriser sa solubilisation. Il s'agit notamment de composés à sulfonater qui sont des polymères d'un sucre, comme le dextrane, substitué par un ou plusieurs groupements carboxylate, dont la protonation conduit à la formation de groupements -COOH, par exemple par passage sur une colonne échangeuse de cations.

Ainsi, les conditions de sulfonation sont suffisamment ménagées et contrôlées pour éviter une décarboxylation du polymère initialement substitué par des groupements carboxyliques. En conséquences, les conditions de sulfonation sont avantageusement les suivantes :

Le polymère à sulfonater est dissous dans un solvant anhydre (voir l'exemple II,1). Un excès molaire de capteur d'acide (comme l'alkène 2M2B) est additionné. Le réactif de sulfonation (S03) comme le complexe S03-DMF est additionné et la réaction se déroule à une température inférieure à la température d'évaporation du capteur d'acide. La réaction est arrêtée par l'ajout d'une solution alcaline, comme le NaHCO3.

Le procédé de l'invention s'applique à des monomères, oligomères ou polymères. Il est tout particulièrement adapté aux polymères comme les HBGFPP ou les RGTA définis précédemment, et donc notamment à des composés dérivés de dextranes ou des copolymères d'acides maliques. Ainsi, l'invention s'intéresse tout spécialement aux polymères de formule :

AaXxYy (I)

dans laquelle :
A est un monomère ; X représente un groupement RCOOR' ; Y représente un groupement O ou N-sulfonate fixé sur A et répondant à l'une des formules suivante ROS03R', RNS03R' ; les groupements R sont des chaînes hydrocarbonées aliphatiques, éventuellement ramifiées et/ou insaturées et qui peuvent contenir un ou plusieurs cycles aromatiques à l'exception de la benzylamine et de la benzylamine sulfonate ; R' représente un atome d'hydrogène ou un cation ; a représente le nombre de monomères ; x représente le taux ou degré de substitution des monomères A par des groupements X ; y représente le taux ou degré de substitution des monomères A par des groupements Y.

Le procédé de l'invention peut être mis en oeuvre sur des polymères, notamment de formule (I), où les liaisons entre les monomères ou entre les monomères et leurs substituants, comme les liaisons A-A ou A-X des polymères de formule (I), sont réalisées par des fonctions instables en milieu acide, notamment des fonctions ester, amide, éther, acétal (osidique) ou autres groupements.

Le procédé de l'invention est donc tout particulièrement adapté pour la préparation de sucres sulfatés, monomériques ou oligomériques, comme certains fragments d'héparine, ou encore pour des polymères de la famille des RGTA notamment lorsque A est un monomère osidique comme dans le cas des polymères à base de dextrane ou de tous autres composés polysaccharidiques.

D'autres avantages et caractéristiques de l'invention apparaîtront des exemples qui suivent concernant la mise en oeuvre du procédé selon l'invention et qui feront références aux dessins en annexe dans lesquels :
- La figure 1 est une représentation graphique d'une molécule de CMDS ou l'aldéhyde terminal est mis en évidence. Pour un CMDS où les valeurs x et y sont différents de 0 il existe 3 types d'unités Z, a) l'aldehyde, ou hémi-acétal, b) le carboxyméthyle glucoside et c) le sulfonate glucoside.
- La figure 2 représente l'équilibre entre la fonction aldéhyde et l'hémi-acétal de l'unité terminale d'un dextrane substitué. L'unité substituée en position anomérique ne participe plus à l'équilibre.
- La figure 3 réprésente le profil chromatographique d'une molécule de type CMDS avec les caractéristiques structurales décrites dans le tableau 3.
- La figure 4 montre les profils chromatographiques obtenus pour un même produit PA 07 sur des colonnes différentes de gel filtration.

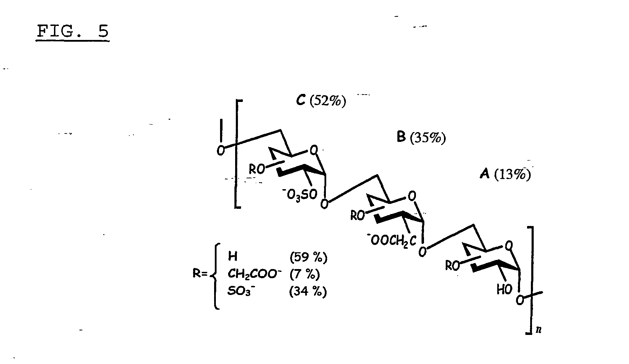

- La figure 5 donne la structure du CMDS.

Ces exemples comprennent aussi la comparaison du procédé de l'invention avec ceux de l'art antérieur, notamment pour la sulfonation de polymères dérivés de dextrane comme les CMDS ou CMDBS. Cette comparaison montre que les protocoles proposés dans les brevets français No. FR 97 15702 et No. FR 9907636 ne permettent pas d'obtenir une bonne préservation de l'intégrité moléculaire des polymères de dextrane car elles génèrent des fragmentations décelables et quantifiables par des méthodes simples ainsi que des hydrolyses de groupements greffés sur les chaînes macromoléculaires.

### I - Evaluation de la sulfonation.

### I.1 - Méthodes de mesure des groupements carboxyliques et sulfonates.

Pour permettre de comparer les différentes méthodes de sulfonation, nous avons mesuré le degré de substitution (ds) en groupements carboxyles (x) et en groupements sulfonates (y) par unité de glucose, Le dextrane étant un polymère de glucose, il possède sur chaque unité de glucose 3 groupements OH réactifs. De ce fait le ds maximal théorique est de 3. Les dosages se font par de méthodes titrimétriques et par analyse élémentaire, selon les protocoles habituels décrits dans l'art antérieur

Le dosage titrimétrique, en combinaison avec l'analyse élémentaire, permet de déterminer les taux de substitution global (x et y) des groupements, carboxyméthyle éther (x) et sulfonates (y).

### I.2 - Détermination de la position des groupements de substitution.

Afin de préciser la position des groupements greffés sur les unités osidiques, nous avons utilisé une méthode d'analyse par RMN (Résonance Magnétique Nucléaire du Proton) selon les conditions habituelles connues de l'homme de l'art (J. Biol. Chem. 275, 38, 29383-29390, 2000). Nous avons utilisé un spectromètre Bruker 200 MHz.

### I.3 - Méthode permettant de mesurer la fragmentation des chaînes poly-osidiques.

Afin de mesurer l'intégrité de la chaîne polymérique avant et après la réaction d'addition des groupements sulfonates, les aldéhydes présents dans le produit en solution ont été dosés.

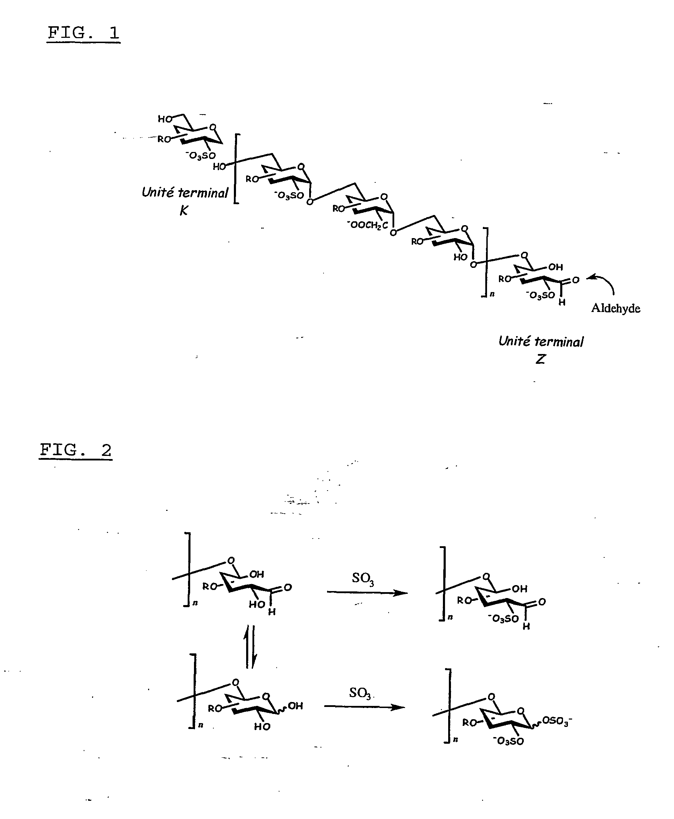

Le dosage des aldéhydes permet de déterminer le nombre d'unités terminales réductrices des produits, puisque seule l'extrémité terminale réductrice du polymère présente un groupement aldéhyde comme montré à la figure 1. Ainsi le nombre de groupement aldéhyde est dosé sur le produit à sulfonater, dans ce cas, il est égal à 1 micromole d'aldéhyde par gramme de produit. Cette valeur doit rester en tous les cas inférieure ou égale à la valeur du produit de départ s'il n'y a pas eu de formation de coupures au cours de la réaction.

En fait la valeur d'aldéhyde libre mesurée après réaction de sulfonation est inférieure à la valeur mesurée avant l'introduction des groupements sulfonate (1,0 micromol/g pour un CMDS à 0,5 mmole/g). Les valeurs d'aldéhyde libre habituellement trouvées pour un produit type CMDS où x = 0,5 et y = 1,25 sont voisines de 0,4 micromoles d'aldéhyde par gramme de produit. Par contre, si ce nombre dépasse la valeur du départ (1,0) il y a forcément formation de coupures.

Cette méthode permet donc de mesurer la formation de fragments car chaque coupure de chaîne génère un nouveau groupe aldéhyde réactif et décelable.

### I.4 - Méthode permettant de mesurer les poids moléculaires et d'étudier l'homogénéité apparente de la préparation.

La mesure du poids moléculaire est réalisée par chromatographie d'exclusion stérique à haute performance en 0,1 M NaNO₃ sur colonnes de gel filtration KB-804 et KB-805 (Shodex Japon) en série à 0,7 ml/min. Les produits sont détectés en sortie de colonne par un détecteur mini Dawn light scattering et un réfractomètre de type RID 10 (J. Biol. Chem. 275, 38, 29383-29390, 2000). L'homogénéité de la préparation est reflétée par la courbe de distribution du poids moléculaire et la mesure de la largeur (à mi-hauteur) du pic ainsi que par la mesure de la symétrie de la courbe.

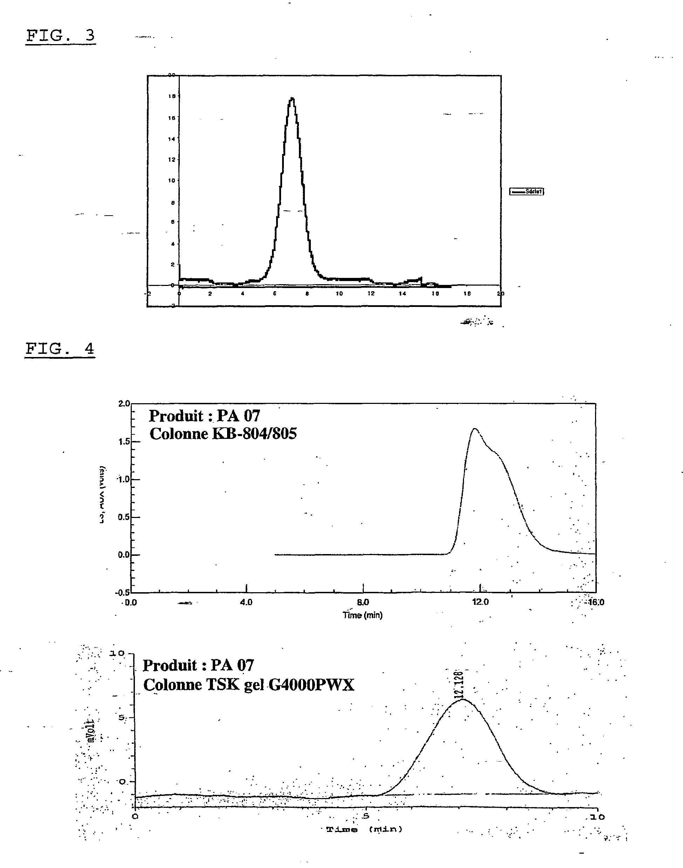

Une deuxième technique de HPLC-Gel filtration permet de montrer l'homogénéité de la molécule en ce qui concerne la répartition des poids moléculaires. Une distribution gaussienne est démontrée sur la figure 3.

Les conditions chromatographiques sont : Colonne : TSKgelG4000PWX(TOSOHAAS) à 30 °C, 7,8 mm ID x 30 cm ; phase mobile : NaCl 0,3 M; débit : 0,7 ml/min ; détection : IR 0,06.

Il a été constaté que pour des produits synthétisés selon les techniques utilisant les complexes de S03-amine l'homogénéité de la préparation varie selon le système chromatographique utilisé.

Quand le produit est séparé par les colonnes Shodex le pic est asymétrique mais quand le deuxième système de séparation chromatographique est utilisé, le pic correspondant au même produit apparaît symétrique comme montré sur la figure 4. Ceci indique que l'homogénéité de la dispersion de poids moléculaires dans ces préparations ainsi que dans celles de l'art antérieur est relative au système de séparation utilisée.

### II - Exemples de O-sulfonation de carboxymethyl dextrane mettant en oeuvre l'invention et comparaison avec l'art antérieur.

Différentes réactions d'O-sulfonation par des réactifs comme l'acide chlorosulfonique, ou bien encore des complexes SO₃-amine, le complexe SO₃-DMF, et le complexe SO₃-DMF en présence du capteur d'acide, le 2-méthyle-2-butène (SO₃-DMF/2m2B), objet de la présente invention sont décrites dans les exemples ci-dessous.

Afin d'établir ces comparaisons, le carboxyméthyl de Dextrane a été choisi comme polymère de départ.

### II.1 - Réaction en présence du capteur d'acide avec le complexe SO₃-DMF/2-méthyle-2-butène.

Dans un ballon de 500 mL, 5 g de carboxyméthyle dextrane forme acide (CMDH⁺), (24,27 mmol) sont dissous dans 40 mL de formamide, puis 40 mL de 2-méthyle-2-butène (25 équivalents) sont additionnés sous agitation. 7,90 g de SO₃-DMF (5 équivalents par unité de glucose) sont additionnés. La réaction se déroule pendant 2 heures à 30°C sous agitation.

La réaction est arrêtée en versant très lentement le milieu réactif dans 200 ml de NaHCO₃ à 2 %. Le pH doit être proche de 7. Si ce n'est pas le cas, la solution est neutralisée par ajout de soude ou de HC1. Dans tous les cas, le pH de la solution ne doit jamais être inférieur à 5 afin d'éviter la dégradation des produits.

Après élimination des excès d'eau, du DMF et du 2M2B par évaporation rotative à pression réduite le produit est purifié par ultrafiltration tangentielle suivie d'une lyophilisation. 6 g de produit sulfonaté sont obtenus sous forme d'une poudre blanche.

Le tableau 1 ci-dessous indique la reproductibilité de l'O-sulfonation du CMDH⁺ (x= 0,52) avec 5 équivalents de SO₃-DMF et 25 équivalents de 2M2B par unité de glucose. Le pourcentage EM (écart à la moyenne) est à voir avec des produits préparés à partir du même CMD.

**Tableau 1**

| | (x) avant sulfonation | COO⁻ en mEq/g (x) après sulfonation | EM % | SO₃⁻ en mEq/g (y) | EM % | Aldéhyde en µmol/g | EM % |
|---|---|---|---|---|---|---|---|
| 1 | 0,52 | 1,58 (0,53) | 2 | 3,82 (1,28) | 5 | 0,35 | 5 |
| 2 | 0,52 | 1,54 (0,50) | 1 | 3,76 (1,25) | 1 | 0,49 | 3 |
| 3 | 0,52 | 1,57 (0,52) | 1 | 3,79 (1,26) | 1 | 0,55 | 3 |
| 4 | 0,52 | 1,52 (0,48) | 2 | 3,64 (1,17) | 1 | 0,60 | 8 |

| | | | | | | | |
|---|---|---|---|---|---|---|---|
| EM = écart à la moyenne (%) | | | | | | | |

Le tableau 2 ci-dessous indique l'O-sulfonation du CMDH⁺ en fonction de la quantité de réactif SO₃-DMF/2M2B (1 :5)

**Tableau 2**

| Equivalents SO₃/DMF(2M2B) par unité glucose | Degré de substitution (ds) | |
|---|---|---|
| | x | y |
| 0,0 | 0,50 | - |
| 2,0 (10,0) | 0,49 | 0,42 |
| 2,5 (12,5) | 0,52 | 0,89 |
| 3,0 (15,0) | 0,52 | 1,00 |
| 4,0 (20,0) | 0,49 | 1,10 |
| 5,0 (25,0) | 0,52 | 1,20 |

Dix manipulations indépendantes ont été effectuées selon ce protocole : Cinq ont été réalisées suivant strictement le même protocole avec 5 équivalents de SO₃-DMF et 25 équivalents de 2M2B, conformément au tableau 1, entrées 1-4. Cinq autres ont été réalisées en faisant varier la quantité de réactif SO₃-DMF/2M2B de 2 à 5 équivalents de réactifs par unité de glucose. Le rapport SO₃-DMF versus 2M2B reste toujours constant 1 : 5 (Tableau 2, entrées, 1-5).

Les résultats montrent que la méthode est reproductible pour l'obtention de produits possédant des groupements éthers et de liaisons osidiques.

Dans tous les cas, le pourcentage EM (écart à la moyenne) est inférieur à la limite maximale fixée à 10 %.

Il convient de noter que (x) ne varie pas significativement (EM <2 %) (il n'y a pas de décarboxylation) et que (y) est < 3%)

Les mesures de poids moléculaires confirment l'absence d'hétérogénéité (figure 3 correspondant au produit décrit dans le tableau 1, entré 4) PM = 67500 +/-7500

Les résultats obtenus démontrent qu'en présence de 2M2B, les taux de sulfonation obtenus sont fonction de la stoechiométrique de la réaction. De plus, les conditions de réaction n'affectent pas le taux des groupes carboxyméthyle éther préalablement greffés sur le CMD.

### II.2 - Définition de la molécule par analyse par RMN du proton.

L'analyse des produits synthétisés a été effectuée en utilisant les techniques analytiques comme le dosage titrimétrique, l'analyse élémentaire, le dosage de sucres réducteurs, et la HPLC/Filtration sur gel couplée à une détection MALLS complétés par la spectroscopie ¹H RMN.

La spectroscopie ¹H RMN permet de préciser la position et le rapport des taux de substitution des différents groupements sur les 3 hydroxyles réactionnels en C-2 et C-3,4 des unités osidiques du dextrane de départ. Le tableau 3 ci-dessous indique la position des substitutions X et Y sur A par analyse RMN dans l'exemple du dextrane de type CM-D-S.

**Tableau 3**

| Polymère | ds | | | Positions des groupes Exprimés dans ds | | | | Poids Moléculaire |
|---|---|---|---|---|---|---|---|---|
| | | | | X | | Y | | |
| | X | Y | OH | C2 | C3+C4 | C2 | C3+C4 | |
| CMDS | 0,50 ± 0,4 | 1,20 ± 0,4 | 1,30 ± 0,4 | 0,35 | 0,15 | 0,52 | 0,68 | 67 000 ± 5 000 |

| | | | | | | | | |
|---|---|---|---|---|---|---|---|---|
| d.s. : Degré de substitution pour chaque groupement par unité de glucose (A). X=CM: CH₂COONa; Y= SO₃Na. OH : représente e nombre de groupement OH n'ayant pas réagi par monomère A. d.s. est calculé à partir des groupements hydroxyle résiduels n'ayant pas été substitués (le nombre OH libre au départ est de 3 par monomère de glucose A. (n=3). C3+C4 : substitution globale dans les positions C-3+C-4, calculé pour X, Y et OH libre comme la différence entre le d.s. total et le d.s. déterminé en position C-2. | | | | | | | | |

### III - Comparaison avec l'art antérieur.

### III.1 - O-Sufonation par l'acide chlorosulfonique. Réaction avec ClSO₃H.

5 g de carboxyméthyle dextrane (24.27 mmol, un équivalent par unité de glucose) sont additionnés dans 162 mL de dichlorométhane. Un mélange hétérogène est obtenu. Le mélange est fortement agité afin d'obtenir une suspension homogène du produit dans le dichlorométhane.

1,6 mL d'acide chlorosulfonique (24.27 mmol, un équivalent par unité de glucose) sont lentement additionnés au mélange. Le milieu réactionnel est maintenu sous agitation pendant 2 heures. Au cours de la réaction, des agglomérats de produits d'une couleur brune se forment. Le mélange est filtré (sur verre fritté n°4) et le produit récupéré est lavé 2 fois avec 100 mL de dichlorométhane, 3 fois avec 100 mL d'un mélange dichlorométhane/dioxane (1:1) et une dernière fois avec 100 mL de dioxane. Le produit obtenu est dissous dans 200 mL d'eau distillée et la solution est amenée à pH 9.5 par addition de soude à 2 M puis à pH 7 par addition d'HCl à-0,05 mol/L.

La solution est filtrée, concentrée et précipitée par 500 mL de méthanol. Le précipité ainsi obtenu est ensuite séché dans une étuve, avant d'être purifié.

Trois manipulations indépendantes ont été effectuées selon ce protocole :
Une avec un équivalent d'acide chlorosulfonique à température ambiante.
Une avec deux équivalents d'acide chlorosulfonique à température ambiante.
Une avec deux équivalents d'acide chlorosulfonique à 4°C.

### III.2 - Réaction avec des complexes SO₃⁻ amine ou amide.

Les protocoles détaillés ci-dessous ont été réalisés en utilisant différents complexes à base de SO₃: le SO₃-Pyridine, le SO₃-triméthylamine, le SO₃-triéthylamine et le SO₃-DMF (Diméthyl Formamide).

Le tableau 4 ci-après indique les caractéristiques structurales et biologiques d e s produits obtenus par O-sulfonation d'un CMD (d.s. C = 0,56) par les différents complexes de SO₃.

Dans un ballon de 500 mL, 5 g de carboxyméthyle dextrane (24,27 mmol, un équivalent par unité de glucose) sont dissous dans 50 mL de formamide. Pour favoriser la dissolution du carboxyméthyle dextrane, la température du milieu réactionnel est apportée vers 50°C. Une fois le produit solubilisé, la solution est ramenée à température ambiante. Pour chaque réaction effectuée, une solution contenant chacun des complexes rapportés dans le tableau 4 ci-dessous est indépendamment préparée dans 50 mL de formamide et additionnée à la solution de carboxyméthyle dextrane sous agitation. La réaction se déroule pendant 2 h à température ambiante.

La réaction est arrêtée en ajoutant 21 d'eau distillée à 4°C. On mélange et on ramène le pH du milieu à7,5-8 à l'aide d'une solution de NaOH 2M.

Après élimination de l'excès d'eau par évaporation rotative à pression réduite le produit est purifié par ultrafiltration tangentielle suivie d'une lyophilisation. 6 g de produit sulfaté sont obtenus sous forme d'une poudre blanche.

Quatre manipulations indépendantes ont été effectuées selon ce protocole :
Une avec deux équivalents de complexe SO₃-pyridine à température ambiante
Une avec deux équivalents de complexe SO₃-triméthylamine à température ambiante
Une avec deux équivalents de complexe SO₃-triéthylamine à température ambiante
Une avec deux équivalents de complexe SO₃-DMF à température ambiante

Par ailleurs une réaction (tableau 4 ligne 8) est réalisée exactement selon le protocole décrit dans l'exemple 5 du brevet français No. FR 97 15702, où la solubilisation du CMD est obtenue par la formation de sels de triéthylammonium. Le SO₃-Pyridine dans un rapport molaire complexe de OH libre est égal 0,4 (ce qui correspond à 2 équivalents de complexe par unité de glucose) est dissous dans du DMSO et ajouté à la solution de polymère. La réaction est faite dans du dimethylformamide à température ambiante.

Le tableau 4 montre les caractéristiques structurales de plusieurs polymères de type CMDS synthétisés selon les différentes méthodes et les contraintes de chaque méthode. Le procédé selon l'invention correspond aux entrés 10 et 11 du tableau 4 et les conditions décrites dans l'exemple N° 1.

**Tableau 4**

| | Réactif de sulfonat ion | Condit ions de réaction | x | y | Aldéhyde terminal (µmol/g) | Activité (*in vivo*) | Toxicité (*in vivo*) |
|---|---|---|---|---|---|---|---|
| 1 | - | - | 0,56 | 0,0 | 1,00 | (-) | (-) |
| 2 | ClSO₃H | 1 ep/22°C | 0,41 | 0,22 | 12,7 | variable | variable |
| 3 | ClSO₃H | 2 eq/22°C | 0,37 | 0,35 | 11,0 | variable | variable |
| 4 | ClSO₃H | 2 eq/4°C | 0,51 | 0,35 | 4,0 | variable | variable |
| 5 | SO₃-Me₃N | 2 eq/22°C | 0,50 | 0,00 | 1,9 | (+) | (+) |
| 6 | SO₃-Et₃N | 2 eq/22°C | 0,29 | 0,40 | 1,6 | (+) | (+) |
| 7 | SO₃-Pyridine | 2 eq/22°C | 0,44 | 0,17 | 1,8 | (+) | (+) |
| 8 | SO₃-Pyridine | 2 eq/22°C | 1, 00 * | 0.35 | 1,9 | (-) | (+) |
| 9 | SO₃-DMF | 2 eq/22°C | 0,37 | 0,23 | 1,5 | Nd | Nd |
| 10 | SO₃-DMF/2M2B | 2 eq/22°C | 0,57 | 0,62 | 0,8 | (+) | (-) |
| 11 | SO₃-DMF/2M2B | 5 eq/22°C | 0,55 | 1,15 | 0,5 | (+) | (-) |

| | | | | | | | |
|---|---|---|---|---|---|---|---|
| Nd = non déterminé * le produit de départ dans cette expérience était substitué en CM à 1 résidu par unité de glucose. | | | | | | | |

Les différents produits de type DCS ont été synthétisés à partir d'un lot unique de dextrane carboxylé (Tableau 4, entrée 1) ayant un degré de substitution en carboxylate de x = 0,56. Après leur purification, les produits ont été caractérisés par les différentes techniques d'analyses structurales.

Les résultats de titrimétrie montrent que les réactions d'O-sulfonation par l'acide chlorosulforique et les complexes de SO₃-amide ou amine provoquent un clivage de la liaison éther des groupes carboxyles greffés (dsC ou x) et une dégradation de la chaîne macromoléculaire (Poids moléculaire et dosage des sucres réducteurs). Ce clivage est d'autant plus marqué que le nombre d'équivalent de réactif ajouté est important. L'abaissement de la température du milieu réactionnel permet de limiter cette dégradation.

Il est à noter que le produit préparé par le complexe SO₃-Et₃N (Tableau 4, entrée 6) est le produit le plus sulfonaté mais aussi celui ayant subit la plus grande décarboxylation. La tendance se confirme avec les autres produits.

Les deux dernières entrées du tableau (entrées 10 et 11) mettent en jeu le produit 2M2B qui agit comme un capteur d'acide afin de limiter la dégradation des liaisons éthers des groupes carboxylates greffés et de la chaîne macromoléculaire et la dégradation de la chaîne.

Il convient de constater qu'en présence du produit 2M2B le dsC du produit obtenu après sulfonation est identique au dsC précurseur. Ceci se vérifie quelle que soit la quantité de réactif additionné. En effet, avec 2.5 fois plus de complexe SO₃-DMF (entrée 11), nous obtenons un dsS supérieur mais surtout le dsC ne change pas. L'efficacité du protocole en présence du 2M2B est ainsi démontrée .

Alors que pour les techniques d'O-sulfonation déjà connues, il y a décarboxylation et dégradation de la chaîne macromoléculaire, la technique d'O-sulfonation en présence du produit 2M2B permet de résoudre le problème de la perte des groupes carboxylates greffés sur le dextrane.

Les mesures d'activités *in vivo* ainsi que celles de toxicité *in vivo* sont réalisées sur des modèles de régénération musculaires du muscle Extensor Digitorum Longus de la patte arrière du rat adulte après écrasement selon les conditions expérimentales décrites dans GAUTRON J., KEDZIA C., HUSMANN I. AND BARRITAULT D. « Accélération de la régénération d'un muscle squelettique de rat adulte par des dérivés de dextranes » C.R. Acad. Sci. Paris, Sciences de la Vie (1995), 318: 671-676. La régénération est quantifiée sur les coupes histologiques par mesures du nombre de fibres musculaires régénérées. La toxicité est mesurée par une diminution de la formation de fibres (comparées aux témoins injectés par une solution de sérum physiologique) ainsi que par l'analyse de l'aspect de la régénération et la mise en évidence de zones anormales de régénération ou la formation de zones inflammatoires montrant une dégénération du tissus musculaire.

### IV - Généralisation du procédé pour d'autres types de polymères.

Le tableau 5 montre la synthèse de plusieurs polymères de formule AaXxYy, où A est un sucre et a est supérieur à 1 par le procédé de l'invention et leurs caractéristiques analytiques.

**Tableau 5**

| | Produit du départ_{dsc} | Échelle | dsS | dsC | Aldéhyde terminal (µmol/g) |
|---|---|---|---|---|---|
| 1 | Dextrane (D) | 2g | 1,19 | - | 0,15 µmol/g |
| 2 | D | 2g | 1,24 | - | 0,15 µmol/g |
| 3 | CM₅₀-D | 2g | 1,22 | 0,5 | 0,20 µmol/g |
| 4 | CM₅₀-Cellulose (C) | 2g | 1,23 | 0,5 | 0,20 µmol/g |
| 5 | CM₅₀-D | 10g | 1,17 | 0,5 | 0,20 µmol/g |
| 6 | CM₅₀-C | 10g | 1,22 | 0,5 | 0,20 µmol/g |
| 7 | CM₆₀-B₆₀-D | 50g | 1,24 | 0,5 | 0,20 µmol/ g |
| 8 | CM₆₀-B₂₀-C | 10g | 1,23 | 0,6 | 0,05 µmol/g |
| 9 | CM₆₀-PhA₆₀-D | 10g | 1,23 | 0,6 | 0,05 µmol/g |
| 10 | CM₆₀-PhA₆₀-C | 10g | 1,22 | 0,6 | 0,05 µmol/g |

## Revendications

1. Procédé de sulfonation de composé présentant une ou plusieurs fonctions hydroxyles libres et/ou une ou plusieurs fonctions amines primaires ou secondaires substituées ou non, **caractérisé en ce qu'**il comprend le traitement dudit composé avec le complexe SO₃-DMF en présence d'un capteur d'acide, choisi dans le groupe comprenant les alkènes ou alkines à point d'ébullition inférieur à 100°C ou un mélange de ceux-ci.

2. Procédé selon la revendication 1 , **caractérisé en ce que** le capteur d'acide est choisi dans le groupe comprenant le 2-méthyle-2-butène (2M2B), le 2-méthyle-propène, le 2-méthyle-pentène, ou leurs isomères, ou un mélange de ceux-ci.

3. Procédé selon la revendication 1 ou 2, **caractérisé en ce qu'**il comprend les étapes suivantes :
a) la solubilisation ou la préparation d'une solution homogène du composé à sulfonater dans un solvant ou un co-solvant anhydre, comme la diméthylformamide (DMF) ou un co-solvant composé de formamide et de diméthylformamide.
b) l'addition à température ambiante (20-22 °C) d'un excès molaire d'un capteur d'acide, comme le 2-méthyle-2-butène, miscible dans le co-solvant.
c) l'addition rapide du complexe SO₃-DMF sous agitation,
d) l'agitation du mélange obtenu à l'étape précédente pendant une à deux heures à une température contrôlée et inférieure à 30 °C,
e) l'arrêt de la réaction par addition progressive du mélange sur une solution alcaline, par exemple une solution à 2 % de bicarbonate acide de soude (NaHCO₃) ou un autre alcali, avec un contrôle de pH jamais inférieur à 4 afin d'obtenir les sels du composé à sulfonater.

4. Procédé selon la revendication 3, **caractérisé en ce qu'**il comprend la purification du composé sulfonaté obtenu à l'étape (e).

5. Procédé selon l'une des revendications 3 ou 4, **caractérisé en ce qu'**il comprend avant l'étape (a), la protonation dudit composé afin de favoriser sa solubilisation lors de l'étape (a).

6. Procédé selon l'une quelconque des revendications précédentes, **caractérisé en ce que** le composé à sulfonater est choisi dans le groupe comprenant les monomères, les oligomères et les polymères.

7. Procédé selon la revendication 6, caratérisé en ce que le composé à sulfonater est un polymère de formule :
AaXxYy (I)
dans laquelle, A est un monomère ; X représente un groupement RCOOR' ; Y représente un groupement O ou N-sulfonate fixé sur A et répondant à l'une des formules suivante ROSO₃R', RNSO₃R' ; les groupements R sont des chaînes hydrocarbonées aliphatiques, éventuellement ramifiées et/ou insaturées et qui peuvent contenir un ou plusieurs cycles aromatiques à l'exception de la benzylamine et de la benzylamine sulfonate ; R' représente un atome d'hydrogène ou un cation ; a représente le nombre de monomères ; x représente le taux ou degré de substitution des monomères A par des groupements X ; y représente le taux ou degré de substitution des monomères A par des groupements Y.

8. Procédé selon l'une des revendications 6 ou 7, **caractérisé en ce que** le polymère est un dérivé du dextrane ou un copolymère d'acides maliques.

9. Procédé selon l'une des revendications 6 à 8, **caractérisé en ce que** le polymère comprend des liaisons entre les monomères ou entre les monomères et leurs substituants réalisées par des fonctions instables en milieu acide, notamment des fonctions ester, amide, éther, acétal.

## Claims

1. Method for sulphonation of compounds comprising one or more free hydroxyl functions and/or one or more primary or secondary substituted or non-substituted amine functions, **characterised in that** it includes the treatment of said compound with the SO₃-DMF complex in the presence of an acid sensor, chosen from the group including alkenes or alkines with a boiling point below 100 °C or a mixture thereof.

2. Method according to claim 1, **characterised in that** the acid sensor is chosen from the group including 2-methyl-2-butene (2M2B), 2-methyl-propene, 2-methyl-pentene, or their isomers, or a mixture thereof.

3. Method according to claim 1 or 2, **characterised in that** it includes the following steps:
a) solubilisation or preparation of a homogeneous solution of the compound to be sulphonated in an anhydrous solvent or co-solvent, such as dimethylformamide (DMF) or a co-solvent composed of formamide and dimethylformamide,
b) addition, at room temperature (20-22 °C), of a molar excess of an acid sensor, such as 2-methyl-2-butene, miscible in the co-solvent,
c) rapid addition of the SO₃-DMF complex under agitation,
d) agitation of the mixture obtained in the previous step for one or two hours at controlled temperature below 30 °C,
e) stopping of the reaction by progressive addition of the mixture to an alkaline solution, for example a solution with 2 % bicarbonate of soda (NaHCO₃) or another alkali, with a pH control never below 4 so as to obtain the salts of the compound to be sulphonated.

4. Method according to claim 3, **characterised in that** it includes the purification of the sulphonated compound obtained in step (e).

5. Method according to one of claims 3 or 4, **characterised in that** it includes, before step (a), the protonation of said compound so as to promote the solubilization in step (a).

6. Method according to any one of the previous claims, **characterised in that** the compound to be sulphonated is chosen from the group including monomers, oligomers and polymers.

7. Method according to claim 6, **characterised in that** the compound to be sulphonated is a polymer of formula:
AaXxYy (I),
in which A is a monomer; X represents an RCOOR' group; Y represents an O or N-sulphonate group bound to A and having one of the following formulas ROSO₃R', RNSO₃R' ; the R groups are aliphatic hydrocarbon chains, possibly branched and/or unsaturated, and can contain one or more aromatic cycles except for benzylamine and benzylamine sulphonate; R' represents a hydrogen atom or a cation; a represents the number of monomers; x represents the level or degree of substitution of A monomers with X groups; and y represents the level or degree of substitution of A monomers with Y groups.

8. Method according to one of claims 6 or 7, **characterised in that** the polymer is a derivative of dextran or a malic acid copolymer.

9. Method according to one of claims 6 to 8, **characterised in that** the polymer includes bonds between the monomers or between the monomers and their substituents created by functions unstable in an acid medium, in particular ester, amide, ether and acetal functions.

## Patentansprüche

1. Verfahren zur Sulfonierung einer Verbindung, eine oder mehrere freie Hydroxylfunktionen und/oder eine oder mehrere primäre oder sekundäre substituierte oder nicht substituierte Aminfunktionen aufweisend, **dadurch gekennzeichnet, dass** es die Behandlung der besagten Verbindung mit dem Komplex SO₃-DMF in Gegenwart eines Säuresensors, ausgewählt aus der Gruppe, die die Alkene oder Alkine bei einem Siedepunkt unter 100 °C oder ein Gemisch derselben, umfasst.

2. Verfahren nach Anspruch 1, **dadurch gekennzeichnet, dass** der Säuresensor aus der Gruppe ausgewählt ist, die das 2-Methyl-2-buten (2M2B), das 2-Methyl-propen, das 2-Methyl-penten oder ihre Isomere oder ein Gemisch derselben umfasst.

3. Verfahren nach Anspruch 1 oder 2, **dadurch gekennzeichnet, dass** es die folgenden Schritte umfasst:
a) die Solubilisierung oder die Zubereitung einer homogenen Lösung der zu sulfonierenden Verbindung in einem Solvens oder einem nicht wässrigem Co-Solvens, wie dem Dimethylformamid (DMF) oder einem Co-Solvens, zusammengesetzt aus Formamin und aus Dimethlyformamin,
b) die Zugabe bei Raumtemperatur (20-22 °C) eines Molüberschusses eines Säuresensors, wie des 2-Methyl-2-butens, mischbar in dem Co-Solvens,
c) die schnelle Zugabe des Komplexes SO₃-DM unter Rühren,
d) das Rühren des im vorangehenden Schritt hergestellten Gemischs während einer bis zwei Stunden bei einer kontrollierten Temperatur und unter 30 °C,
e) das Anhalten der Reaktion durch schrittweise Zugabe des Gemischs auf eine alkalische Lösung, zum Beispiel eine Lösung mit 2 % Natriumhydrogencarbonat ((NaHCO₃) oder ein anderes Alkali, mit einer pH-Kontrolle nie unter 4, um die Salze der zu sulfonierenden Verbindung herzustellen.

4. Verfahren nach Anspruch 3, **dadurch gekennzeichnet, dass** es die Reinigung der in Schritt (e) hergestellten sulfonierten Verbindung umfasst.

5. Verfahren nach einem der Ansprüche 3 oder 4, **dadurch gekennzeichnet, dass** es vor dem Schritt (a) die Protonierung der besagten Verbindung umfasst, um ihre Solubilisierung bei Schritt (a) zu fördern.

6. Verfahren nach einem der vorangehenden Ansprüche, **dadurch gekennzeichnet, dass** die zu sulfonierende Verbindung aus der Gruppe ausgewählt ist, die die Monomere, die Oligomere und die Polymere umfasst.

7. Verfahren nach Anspruch 6, **dadurch gekennzeichnet, dass** die zu sulfonierende Verbindung ein Polymer der Formel
AaXxYy (I)
ist, in der A ein Monomer ist, X eine RCOOR'-Gruppe darstellt, Y eine Gruppe O oder N-Sulfonat gebunden auf A darstellt und einer der folgenden Formeln ROSO₃R', RNSO₃R' entspricht, die R-Gruppen aliphatische Kohlenwasserstoffketten sind, eventuell verzweigt und/oder ungesättigt und die einen oder mehrere aromatische Ringe enthalten können mit Ausnahme von Benzylamin und Benzylaminsulfonat, R' ein Wasserstoffatom oder ein Kation darstellt, a die Monomeranzahl darstellt, x den Anteil oder den Grad der Substitution der A-Monomere durch X-Gruppen darstellt, y den Anteil oder den Grad der Substitution der A-Monomere durch Y-Gruppen darstellt.

8. Verfahren nach einem der Ansprüche 6 oder 7, **dadurch gekennzeichnet, dass** das Polymer ein Derivat des Dextrans oder ein Copolymer von Apfelsäuren ist.

9. Verfahren nach einem der Ansprüche 6 bis 8, **dadurch gekennzeichnet, dass** das Polymer Bindungen - zwischen den Monomeren oder zwischen den Monomeren und ihren Substituenten umfasst, durchgeführt von in saurem Milieu instabilen Funktionen, vor allen von Ester-, Amid-, Ether-, Acetalfunktionen.
